(19) Europäisches Patentamt
European Patent Office
Office européen des brevets

(11) Numéro de publication: **0 259 228 B1**

(12) **FASCICULE DE BREVET EUROPEEN**

(45) Date de publication de fascicule du brevet: **03.06.92**

(51) Int. Cl.[5]: **C07D 263/20**, A61K 31/42

(21) Numéro de dépôt: **87401965.6**

(22) Date de dépôt: **02.09.87**

(54) **Dérivés 5-aminoéthylés de l'oxazolidinone-2, leur procédé de préparation et leur application en thérapeutique.**

(30) Priorité: **03.09.86 FR 8612369**
**30.12.86 FR 8618368**

(43) Date de publication de la demande:
**09.03.88 Bulletin 88/10**

(45) Mention de la délivrance du brevet:
**03.06.92 Bulletin 92/23**

(84) Etats contractants désignés:
**BE CH DE ES FR GB GR IT LI LU NL SE**

(56) Documents cités:
**FR-A- 2 428 032**
**FR-A- 2 500 450**
**FR-A- 2 528 046**

(73) Titulaire: **DELALANDE S.A.**
**32, rue Henri Regnault**
**F-92400 Courbevoie(FR)**

(72) Inventeur: **Langlois, Michel**
**4, place César Franck**
**F-78530 BUC(FR)**
Inventeur: **Schoofs, Alain René**
**242, rue de la Croix-Nivert**
**F-75015 Paris(FR)**
Inventeur: **Rumigny, Jean François**
**7, boulevard Richelieu**
**F-92500 Rueil Malmaison(FR)**
Inventeur: **Dostert, Philippe**
**10, Place des Vosges**
**F-75004 Paris(FR)**
Inventeur: **Strolin-Benedetti, Margherita**
**10, Place des Vosges**
**F-75004 Paris(FR)**
Inventeur: **Renaut, Patrice**
**3, ruelle de Plombières Hauteville**
**F-21121 Fontaine les Dijon(FR)**

(74) Mandataire: **Kedinger, Jean-Paul et al**
**c/o Cabinet Malemont 42, avenue du Président Wilson**
**F-75116 Paris(FR)**

EP 0 259 228 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés de l'oxazolidinone-2, leur procédé de préparation et leur application en thérapeutique.

Par FR-A-2 500 450, on connait déjà notamment des composés racémiques de formule :

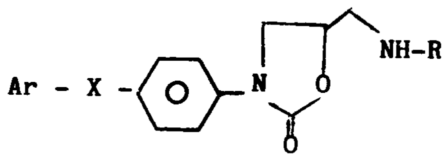

dans laquelle :

- R = H ou alkyle en $c_1$ - $c_4$,
- Ar = phényle, phényle substitué par un atome d' halogène ou par un groupe $CF_3$ ; ou (chloro-3 fluoro-4) phényle ; et
- X représente - $CH_2$-O- .

Par FR-A-2 528 046, on connaît également le composé de formule :

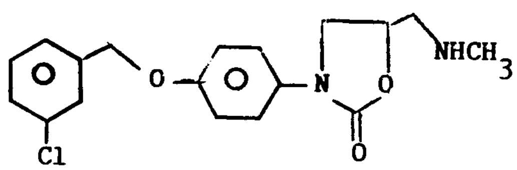

[R(+)]

Les nouveaux dérivés selon l'invention sont tels que définis à la revendication 1.

On ajoutera que le symbole A utilisé dans ce qui suit, correspond à la configuration relative érythro ; les composés IA(-) sont donc de configuration relative érythro (-) et les composés IA (+) sont de configuration relative érythro (+). Les couples racémiques érythro correspondants sont symbolisés IA(+), IA(-) ou encore IA(±). Comme indiqué plus loin, les composés IA(-) sont de configuration absolue (R, S) et les composés IA-(+) sont de configuration absolue (S, R).

Le procédé selon l'invention pour la préparation des composés de formule (I) est comme suit :

Dans une première étape, on condense les anilines de formule :

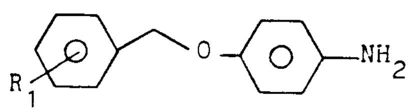

(II)

où $R_1$ a la même signification que dans la formule (I), respectivement avec le mélange des 4 stéréoisomères de l'époxy-1,2 butanol-3 de formule :

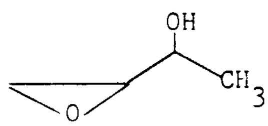

(III)

cette condensation étant de préférence effectuée à chaud dans un solvant organique, en particulier à reflux dans un solvant organique hydroxylé tel que l'isopropanol.

Le mélange des 4 stéréoisomères de formule (III) est quant a lui obtenu par époxydation du butène-3,4 ol-2 racémique de formule :

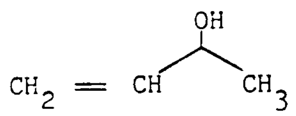

(IV)

Pour effectuer cette époxydation, on fait appel à un agent d'époxydation tel que l'acide méta-chloroperbenzoïque et on opère de préférence à température ambiante dans un solvant organique tel que le chlorure de méthylène.

Dans une seconde étape, on soumet chaque mélange de 4 stéréoisomères de formule (V), obtenu a la première étape :

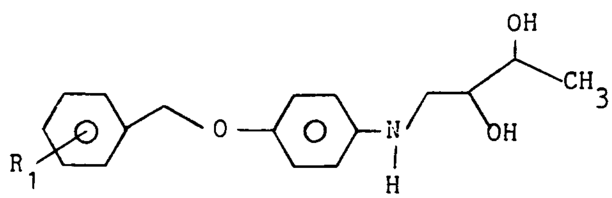

(V)

où $R_1$ a la même signification que dans la formule (I), à une cyclisation par action du carbonate d'éthyle en présence d'éthylate de sodium, de préférence à chaud dans un solvant organique, en particulier à reflux dans le toluène, ce qui conduit à un mélange de 4 stéréoisomères de formule :

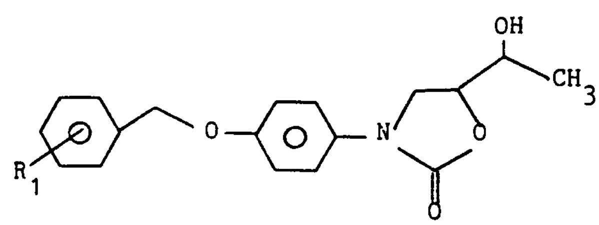

(VI)

où $R_1$ a la même signification que dans la formule (I).

Dans une troisième étape, on soumet chaque mélange de 4 stéréoisomères de formule (VI), à une chromatographie sur colonne de silice sous moyenne pression, ce qui permet de séparer ledit mélange en deux couples de diastéréoisomères racémiques, à savoir un couple (le moins polaire) de configuration relative érythro VI A(+)/VI A(-) [ou VI A(±)] et un couple (le plus polaire) de configuration relative thréo VI B-(+)/VI B(-) [ou VI B(±)].

Dans une quatrième étape, on dédouble chaque couple VI A(±) et VI B(±) pour isoler les énantiomères correspondants.

Le dédoublement du couple érythro VI A(±) peut par exemple être réalisé par estérification à l'aide du chlorure de l'acide phénoxy-2 propionique (+) (VII) dans la pyridine, suivant le schéma suivant :

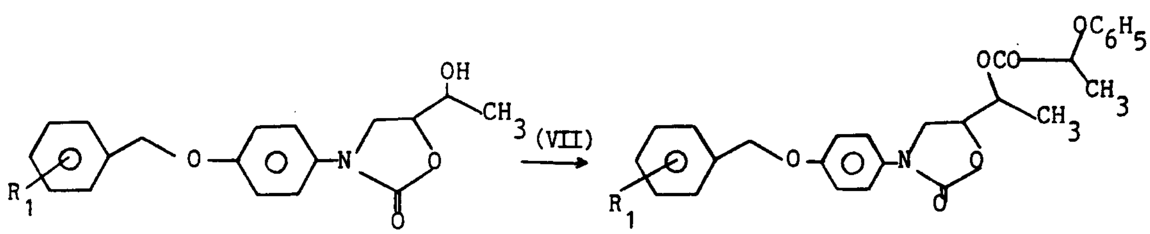


VIA(±) VI'A

cette estérification étant suivie d'une séparation par chromatographie sur colonne de silice sous moyenne pression, du couple de diastéréoisomères érythro VI' A, ce qui conduit d'une part à l'énantiomère ester le

moins polaire VI′ A(+) et d'autre part à l'énantiomère ester le plus polaire VI′ A(-). Les deux énantiomères ester VI′ A(+) et VI′ A(-) ainsi isolés sont ensuite saponifiés, par exemple à la soude, pour obtenir respectivement l'énantiomère VI A(+) et l'énantiomère VI A(-).

Quant au dédoublement du couple thréo VI B(±), il peut être réalisé par estérification à l'aide du chlorure de l'acide α-méthoxy α-trifluorométhylphényl acétique (-) (VIII) dans la pyridine suivant le schéma :

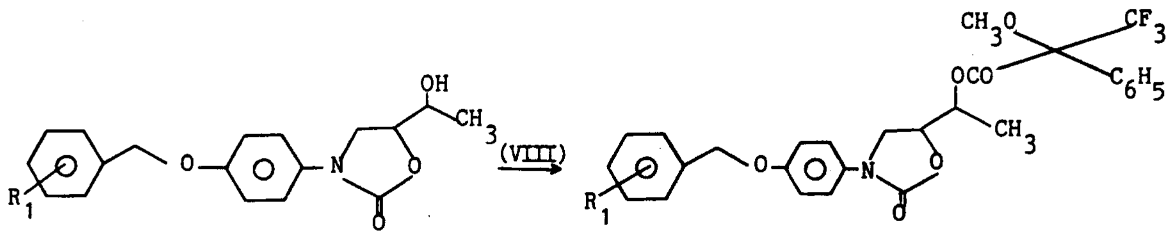


VIB(±) VI"B

cette estérification étant suivie d'une séparation par chromatographie sur colonne de silice sous moyenne pression, du couple de diastéréoisomères thréo VI″ B, ce que conduit d'une part à l'énantiomère ester le moins polaire VI″ B(+) et d'autre part à l'énantiomère ester le plus polaire VI″ B(-). Les deux énantiomères esters VI″ B(+) et VI″ B(-) ainsi isolés sont ensuite saponifiés, par exemple à la soude, pour obtenir respectivement l'énantiomère VI B(+) et l'énantiomère VI B(-).

Il convient par ailleurs de préciser ici que la configuration absolue de chacun des énantiomères VI A-(+), VI A(-), VI B(+) et VI B(-) a été déterminée par la méthode du dédoublement cinétique partiel de l'anhydride α-phénylbutyrique par ces énantiomères (Weidmann R., Schoofs A.R. et Horeau A., Comptes Rendus Acad. Sci., Paris, Série II, 1984, page 319 et références citées). Ainsi :

- à l'énantiomère VI A(+) a pu être attribuée la configuration absolue (S, R),
- à l'énantiomère VI A(-) a pu être attribuée la configuration absolue (R, S),
- à l'énantiomère VI B(+) a pu être attribuée la configuration absolue (S, S), et
- à l'énantiomère VI B(-) a pu être attribuée la configuration absolue (R, R).

Dans une cinquième étape, on forme respectivement le mésylate des stéréoisomères VI A(+), VI A(-), VI B(+) et VI B(-), notamment par réaction d'un halogénure de mésyle sur ces stéréoisomères, les mésylates résultants répondant à la formule :

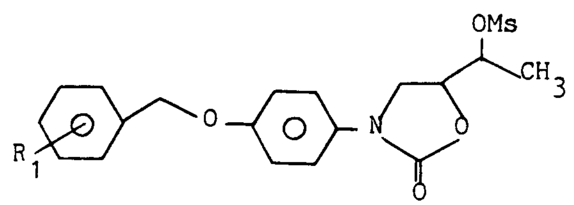

[IXA(+), IXA(-), IXB(+) ou IXB(-)]

où $R_1$ a la même signification que dans la formule (I) et Ms est le groupe mésyle, cette réaction étant effectuée en présence d'une base organique telle que la triéthylamine, de préférence dans un solvant organique notamment un solvant organique halogéné tel que le chlorure de méthylène.

Dans la sixième et dernière étape, chaque stéréoisomère mésylate obtenu à l'étape précédente est mis à réagir, éventuellement en présence d'un solvant organique et en particulier un solvant organique hydroxylé tel que l'éthanol ou l'isopropanol, avec un composé de formule :

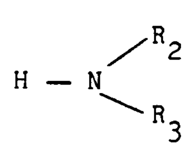

(X)

où le couple ($R_2$, $R_3$) a la même signification que dans la formule (I), ce qui conduit au stéréoisomère

correspondant de formule (I). Il est à remarquer que la substitution nucléophile par le composé (X) se produit avec inversion de configuration. Plus précisément :

- à partir du stéréoisomère mésylate IX A(+) de configuration absolue (S, R), on obtient le stéréoisomère thréo (+) [I B(+)] de configuration absolue (S, S),
- à partir du stéréoisomère mésylate IX A(-) de configuration absolue (R, S), on obtient le stéréoisomère thréo (-) [I B(-)] de configuration absolue (R, R),
- à partir du stéréoisomère mésylate IX B(+) de configuration absolue (S, S), on obtient le stéréoisomère érythro (+) [I A(+)] de configuration absolue (S, R), et
- à partir du stéréoisomère mésylate IX B(-) de configuration absolue (R, R), on obtient le stéréoisomère érythro (-) [I A(-)] de configuration absolue (R, S).

Les composés de formule (I) peuvent en variante être préparés de la manière suivante.

On répète les quatre premières étapes définies précédemment, en utilisant à titre de produit de départ, le composé de formule :

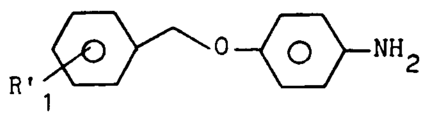

(IIa)

où $R'_1$ représente une seule des significations données pour $R_1$ dans la formule (I), ce qui conduit aux 4 stéréoisomères de formule :

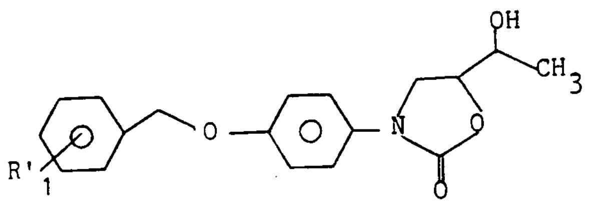

VIaA(+) (S,R)
VIaA(-) (R,S)
VIaB(+) (S,S)
VIaB(-) (R,R)

où $R'_1$ a la même signification que dans la formule (IIa).

Ces 4 stéréoisomères sont ensuite :

- soit traités respectivement comme dans les cinquième et sixième étapes décrites précédemment, ce qui conduit aux 4 stéréoisomères de formule :

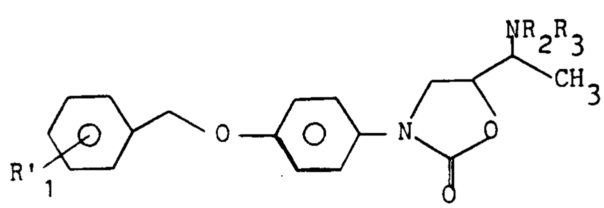

IaB(+) (S,S)
IaB(-) (R,R)
IaA(+) (S,R)
IaA(-) (R,S)

  où $R'_1$ a la signification unique donnée pour la formule (IIa) et le couple ($R_2$, $R_3$) a les mêmes significations que dans la formule (I),
- soit soumis respectivement à une débenzylation, ce qui conduit aux 4 stéréoisomères de formule :

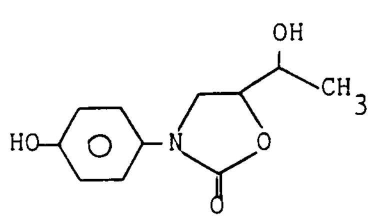

XIA(+) (S,R)
XIA(-) (R,S)
XIB(+) (S,S)
XIB(-) (R,R)

Cette débenzylation peut en particulier consister en une hydrogénolyse en présence d'un catalyseur d'hydrogénolyse tel que le palladium sur charbon, de préférence dans un solvant organique tel qu'un solvant organique hydroxylé comme l'éthanol, sous atmosphère d'hydrogène.

Cette débenzylation est suivie par un alkylation de la fonction phénolique des stéréoisomères XI A(+) (S, R), XI A(-) (R, S), XI B(+) (S, S) et XI B(-) (R, R), par un composé de formule :

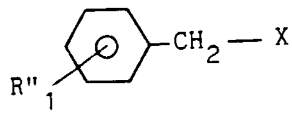

(XII)

où $R''_1$ a les mêmes significations que $R_1$ dans la formule (I), à l'exception de la signification de $R'_1$ donnée pour la formule (IIa) et X est un groupe bon partant tel qu'un atome d'halogène. Cette alkylation est de préférence réalisée dans un solvant organique, en particulier un solvant organique polaire tel que l'acétonitrile, le diméthylformamide, le diméthylsulfoxyde ou la butanone-2, en présence d'une base minérale en particulier le carbonate de potassium.

De cette alkylation résultent respectivement les 4 stéréoisomères de formule :

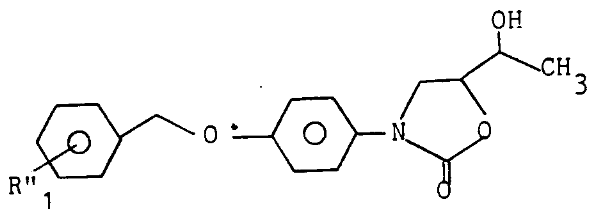

XIIIA(+) (S,R)
XIIIA(-) (R,S)
XIIIB(+) (S,S)
XIIIB(-) (R,R)

où $R''_1$ a les mêmes significations que dans la formule (XII).

Ces 4 derniers stéréoisomères sont ensuite traités respectivement comme dans les cinquième et sixième étapes décrites précédemment, ce qui conduit aux 4 stéréoisomères de formule :

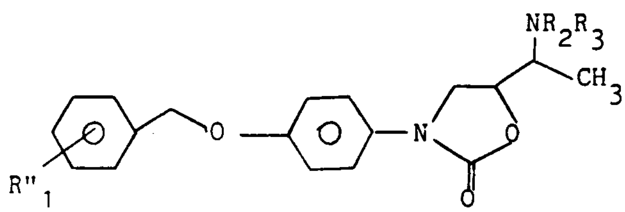

IbB(+) (S,S)
IbB(-) (R,R)
IbA(+) (S,R)
IbA(-) (R,S)

où $R''_1$ a les mêmes significations que dans la formule (XII) et le couple ($R_2$, $R_3$) a les mêmes significations que dans la formule (I).

Il est à noter que dans ce qui précède et ce qui suit, lorsque la configuration absolue des composés selon l'invention est symbolisée par (S, R), cela signifie que le carbone asymétrique du cycle oxazolidinone possède la configuration S et l'autre carbone asymétrique la configuration R. De la même manière, lorsque la configuration absolue des composés selon l'invention est symbolisée par (R, S), cela signifie que le carbone asymétrique du cycle oxazolidinone est de configuration R et l'autre carbone asymétrique de configuration S.

Les préparations suivantes sont données à titre d'exemples pour illustrer l'invention.

Exemple 1 : Synthèse du mélange des 4 stéréoisomères de l'époxy-1,2 butanol-3 [III] (numéro de code 790255)

A une solution refroidie à 0 ° C de 83,6 g (0,48 mole) d'acide méta-chloroperbenzoïque dans 250 ml de $CH_2Cl_2$ est ajoutée goutte à goutte une solution de 33,3 g (0,46 mole) de butène-3,4 ol-2 dans 100 ml de $CH_2Cl_2$. Après 20 heures d'agitation à 20 ° C, le milieu partiellement gélifié est refroidi à -18 ° C puis filtré. La phase organique est brassée pendant 30′ en présence de 10 g de $Na_2CO_3$ solide. Après filtration et évaporation sous très léger vide, on récupère 36 g d'une huile jaune clair (rendement : 88 %) correspondant

au mélange attendu et composée de 65 % d'isomère thréo et 35 % d'isomère érythro.

Exemple 2 : Synthèse du mélange des 4 stéréoisomères du [(chloro-3 benzyloxy)-4 phénylamino]-1 butane diol-2,3 [V] (numéro de code 340245)

Une solution contenant 50 g (0,21 mole) de 4-(chloro-3′-benzyloxy) aniline et 21,6 g (0,24 mole) du mélange obtenu à l'exemple 1, dans 850 ml d'isopropanol est portée à reflux pendant 6 h 30. Après évaporation de l'isopropanol, le mélange brut est purifié par chromatographie flash sur silice (éluant : $CH_2Cl_2$-$CH_3OH$ : 97-3). On récupère 34 g (rendement : 49 %) de solide blanc correspondant au produit attendu et ayant un point de fusion de 95-96° C.

Exemple 3: Préparation du mélange des 4 stéréoisomères de la [(chloro-3 benzyloxy)-4 phényl]-3 (hydroxy-1 éthyl)-5 oxazolidinone-2 [VI]

A partir d'une solution de 600 ml de toluène contenant 33,4 g (0,10 mole) du mélange obtenu à l'exemple 2 sont distillés 150 ml de solvant sous atmosphère d'argon. On ajoute ensuite successivement 15 g (15,5 ml ; 0,127 mole) de carbonate d'éthyle puis 3,4 ml d'une solution de EtONa dans EtOH anhydre 1M. Après 4 h 30 à reflux, la réaction de cyclisation est complète. Le milieu réactionnel est concentré puis repris par 300ml de méthyl éthyl cétone. Après lavage avec HCl 2N, puis avec une solution saturée de NaCl, on récupère 35 g de solide brun correspondant au produit attendu.

Exemple 4 : Séparation des deux couples de diastéréoisomères racémiques [VIA(±) et VIB(±)] du [(chloro-3 benzyloxy)-4 phényl]-3 (hydroxy-1 éthyl)-5 oxazolidinone-2 [VI]

On soumet à une chromatographie sous moyenne pression (éluant : acétate d'éthyle - heptane : 70-30), le mélange obtenu à l'exemple 3 et on isole ainsi 2 produits à mobilité différente sur plaque de $SiO_2$ :

- le produit le plus mobile (le moins polaire) - 11,4 g d'un solide blanc ayant un point de fusion de 88° C - étant constitué par le diastéréoisomère racémique de configuration relative érythro [VIA(±) ; numéro de code : 300868], et
- le produit le moins mobile (le plus polaire) - 18,7 g d'un solide blanc ayant un point de fusion de 114,5° C - étant constitué par le diastéréoisomère racémique de configuration relative thréo [VIB(±) ; numéro de code 300869].

Exemple 5 : Dédoublement du diastéréoisomère racémique thréo [VIB(±) ; numéro de code : 300869] du [-(chloro-3 benzyloxy)-4 phényl]-3 (hydroxy-1 éthyl)-5 oxazolidinone-2 [VI].

Dans un ballon sous argon contenant 9 g (0,035 mole) du chlorure de l'acide α-méthoxy α-trifluorométhylphénylacétique {$[\alpha]_D^{20}$ = - 134°6 (C=5,2 ; $CCl_4$)] et 30 ml de pyridine, sont additionnés à 0° C en plusieurs fois 11,2 g (0,032 mole) du couple VIB(±) obtenu à l'exemple 4. Après solubilisation à l'aide de 25 ml de pyridine, 1,2 g (0,014 mole) de 4-diméthylaminopyridine sont ajoutés. Au bout de 60 heures d'agitation à température ambiante, la réaction est complète. On verse sur 50 ml d'eau, puis extrait 3 fois à l'ether. La phase organique est lavée 3 fois avec HCl 2N, puis par une solution saturée de $NaHCO_3$, séchée sur $MgSO_4$ et évaporée.

Le mélange brut obtenu est chromatographié sur colonne de silice sous moyenne pression (éluant = acétate d'éthyle-hexane : 50-50) pour donner les deux esters de MOSHER séparés VI″B(+) et VI″B(-) sous forme d'huile :

- énantiomère ester VI″B(+) [le plus mobile sur plaque $SiO_2$]: 7,9 g ; $[\alpha]_D^{20}$ = + 25,3° (C = 1,0 ; $CH_2Cl_2$)
- énantiomère ester VI″B(-) [le moins mobile sur plaque $SiO_2$]: 7,6g ; $[\alpha]_D^{20}$ = - 73,7° (C = 1,1 ; ($CH_2Cl_2$)

A une solution refroidie à - 10° C de 3,6 g (0,0064 mole) de l'énantiomère ester VI″B(+) dans 50 ml de $CH_3OH$ sont additionnés 4,8 ml de NaOH 2N. La saponification est complète après 8 heures d'agitation à 20° C. Après concentration à froid, le milieu réactionnel est étendu par 160 ml de méthyl éthyl cétone et 50 ml de mélange glace-eau. Aprés décantation, la phase organique est lavée par une solution de NaCl, séchée sur $MgSO_4$, puis évaporée. Par chromatographie flash (éluant = acétate d'éthyl - hexane : 70-30), on récupère 2 g (rendement = 90 %) d'un solide blanc (F = 90° C) qui est l'énantiomère thréo (+) [VIB-(+) ; numéro de code : 300872] du [(chloro-3 benzyloxy)-4 phényl]-3 (hydroxy-1 éthyl)-5 oxazolidinone-2 :

. $[\alpha]_D^{20}$ = + 39,5° (C = 1,03 ; $CH_2Cl_2$)

. configuration absolue : (S,S).

La saponification, dans les mêmes conditions que ci-dessus, de l'énantiomère ester VI″B(-), conduit à l'énantiomère thréo (-) [VIB(-) ; numéro de code : 300873] du [(chloro-3 benzyloxy)-4 phényl]-3 (hydroxy-1 éthyl)-5 oxazolidinone-2 :

. Point de fusion : 90° C
. $[\alpha]_D^{20}$ = -41,5° (C = 1,01 ; $CH_2Cl_2$)
. Configuration absolue : (R,R).

Exemple 6 : Dédoublement du diastéréoisomère racémique érythro [VIA(±) ; numéro de code : 300868] du [(chloro-3 benzyloxy)-4 phényl]-3 (hydroxy-1 éthyl)-5 oxazolidinone-2[VI].

A une solution refroidie à 0° C de 4,25 g (0,023 mole) du chlorure de l'acide phénoxy-2 propionique {-$[\alpha]_D^{20}$ = + 26,3° (C = 1,0 ; $CH_2Cl_2$)} et 0,65 g (0,00053 mole) de 4-diméthylaminopyridine dans 50 ml de pyridine, sont additionnés en 30′ 6,15 g du couple VIA(±) obtenu à l'exemple 4, dissous dans 30 ml de pyridine. La réaction d'estérification est complète aprés 16 heures d'agitation à 20° C. Le milieu réactionnel est traité comme lors de l'estérification de l'exemple 5. Par chromatographie moyenne pression (éluant = acétate d'éthyle - hexane : 40-60), on récupère :

- l'énantiomère ester VI′A(+) [le plus mobile sur plaque de $SiO_2$] : 3,51 g ; $[\alpha]_D^{20}$ = + 37,6° (C = 1,0 ; $CH_2Cl_2$) ; et
- l'énantiomère ester VI′A(-) [le moins mobile sur plaque de $SiO_2$] : 3,66 g ; $[\alpha]_D^{20}$ = -14,2° (C = 1,0 ; $CH_2Cl_2$).

La saponification par NaOH 2N (1,3 éq.) de ces énantiomères ester pendant 2 heures à 20° C dans les mêmes conditions opératoires que dans l'exemple 5, suivie d'une extraction classique à l'acétate d'éthyle permet d'isoler respectivement :

- l'énantiomère érythro (+) [VIA(+) ; numéro de code : 340177] du [(chloro-3 benzyloxy)-4 phényl]-3 (hydroxy-1 éthyl)-5 oxazolidinone-2 :
  . Point de fusion : 78° C
  . $[\alpha]_D^{20}$ = + 16,6° (C = 1,0 ; $CH_2Cl_2$)
  . Configuration absolue : (S, R) ; et
- l'énantiomère érythro (-) [VIA(-) ; numéro de code : 340176] du [(chloro-3 benzyloxy)-4 phényl]-3 (hydroxy-1 éthyl)-5 oxazolidinone-2 :
  . Point de fusion : 78° C
  . $[\alpha]_D^{20}$ = - 16,2° (C = 1,0 ; $CH_2Cl_2$)
  . Configuration absolue : (R, S).

Par mise en oeuvre des procédés objet des exemples 1 à 6 ci-dessus, mais en partant des réactifs appropriés, on obtient les autres composés (VI).

Le tableau 1 ci-après rassemble les données physico-chimiques des énantiomères préparés aux exemples 5 et 6.

Exemple 7 : Préparation du stéréoisomère thréo (-) (R,R) mésylate de [(chloro-3 benzyloxy)-4 phényl]-3 (hydroxy-1 éthyl)-5 oxazolidinone-2 [IXB(-) ; numéro de code 340270]

Dans un ballon de 500 ml contenant 15 g (43 mmoles) de l'énantiomère de numéro de code 300873 préparé à l'exemple 5, en solution dans 150 ml de $CH_2Cl_2$ sont versés 7,4 g (5 ml, 65 mmoles) de chlorure de mésyle. Après refroidissement à - 10° C, sont additionnés goutte à goutte 6,5 g (9 ml, 65 mmoles) de triéthylamine sous atmosphère d'argon.

Après une heure d'agitation à température ambiante, le milieu réactionnel est versé sur un mélange eau-glace et extrait 3 fois au $CH_2Cl_2$ (150 ml). Les phases organiques sont lavées par une solution saturée de $NaHCO_3$ (50 ml) puis une solution saturée de NaCl (2 x 50 ml).

Après séchage sur $MgSO_4$, filtration et évaporation sous pression réduite, on récupère 18 g de solide blanc correspondant au stéréoisomère attendu:

. Point de fusion : 117° C
. $[\alpha]_D^{20}$ = - 45,2° (C = 1 ; $CH_2Cl_2$)

Exemple 8 : Préparation du stéréoisomère érythro (-) (R, S) [(chloro-3 benzyloxy)-4 phényl]-3 [-(diméthylamino-1 éthyl)]-5 oxazolidinone-2 [IA(-) : numéro de code 200562]

Une "bombe" en acier inoxydable contenant 8,2 g (19,5 mmoles) du mésylate obtenu à l'exemple 7 et

30 ml (≃ 0,45 mole) de diméthylamine est chauffée à 100° C.

Aprés 4 heures d'agitation sous une pression de $4.10^5$ Pa, le milieu réactionnel est concentré.

Le précipité récupéré est purifié par chromatographie flash sur colonne de silice (éluant : $CH_2Cl_2$-$CH_3OH$ : 96-4).

On récupère ainsi 4,2 g d'un produit incolore correspondant au produit attendu.

Exemple 9 : Préparation de l'énantiomère thréo (-) (R,R) de l'[hydroxy-4 phényl]-3 (hydroxy-1 éthyl)-5 oxazolidinone-2 [XIB(-) ; numéro de code : 340315]

A une fiole de 1 litre contenant 4 g de Pd/C à 10 % en suspension dans 350 ml d'ethanol à 95 % sont introduits 39,3 g (0,125 mole) de l'énantiomère de numéro de code 300873 préparé à l'exemple 5. Après 2 heures d'agitation forte sous atmosphère d'hydrogène, la réaction d'hydrogénolyse est complète. Le catalyseur est récupéré par filtration et le filtrat est évaporé sous pression réduite pour laisser une huile jaune clair qui cristallise. Un brasssage d'une heure dans le pentane donne 27 g (rendement : 97 %) d'un solide blanc correspondant au produit attendu :

. Point de fusion : 149° C
. $[\alpha]_D^{20}$ = - 73,2° (C = 1,0 ; $CH_3OH$)

Les autres énantiomères de l'[hydroxy-4 phényl]-3 (hydroxy-1 éthyl)-5 oxazolidinone-2 sont obtenus par un processus analogue à partir des réactifs appropriés et sont répertoriés dans le tableau 3 ci-après.

Exemple 10 : Préparation de l'énantiomère thréo (R,R) (-) [(méthoxy-3 benzyloxy)-4 phényl]-3 (hydroxy-1 éthyl)-5 oxazolidinone-2 [I ; numéro de code : 200436]

Dans un ballon surmonté d'un réfrigérant sont introduits, successivement, 1 g (0,0045 mole) de (hydroxy-4 phényl)-3 (hydroxy-1 éthyl)-5 oxazolidinone-2 [XI] (numéro de code 340315), 15 ml d'acétonitrile, 1,2 g (0,0089 mole) de $K_2CO_3$ finement broyé, 0,1 g de KI et 0,75 ml (0,77 g, 0,005 mole) de méthoxy-3 chlorométhyl-1 benzène. Après 3 heures d'agitation sous reflux, les minéraux sont séparés par filtration sur verre fritté, le filtrat est étendu par 150 ml d'eau. La phase aqueuse décantée est épuisée par $CH_2Cl_2$ (2 x 50 ml). La phase organique est lavée par une solution saturée de NaCl, séchée sur $MgSO_4$ et filtrée, puis évaporée. Le solide blanc récupéré est purifié par chromatographie flash de silice (éluant : $CH_2Cl_2$-$CH_3OH$ : 96-4). Après évaporation, on récupère 0,93 g du produit attendu (cristallisé, incolore).

. Rendement : 62%
. Point de fusion : 116°C
. $[\alpha]_D^{20}$ = -41,9° (C = 1 ; $CH_2Cl_2$)
. Analyse élémentaire pour $C_{19}H_{21}NO_5$ : 342,366
Calc.(%) C 66,46 H 6,16 N 4.08
Tr. (%) 66,71 6,43 4,30

Par mise en oeuvre des procédés exemplifiés ci-dessus, mais à partir des réactifs appropriés, on obtient les autres composés (I) dont certains sont repertoriés dans le tableau 2 ci-après.

TABLEAU 1

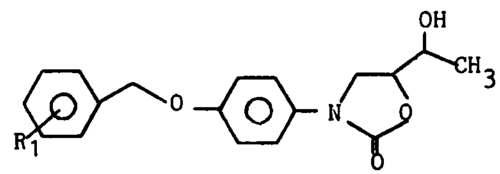

[VI]

| Numéro de Code | $R_1$ | Configuration absolue | $[\alpha]_D^{20}$ (C = 1 ; $CH_2Cl_2$) | Formule brute | Poids moléculaire | Point de fusion (°C) | ANALYSE ELEMENTAIRE % | C | H | N |
|---|---|---|---|---|---|---|---|---|---|---|
| 300873 | 3-Cl | (R, R) | - 41,5° | $C_{18}H_{18}ClNO_4$ | 347,789 | 90 | Cal. | 62,16 | 5,22 | 4,03 |
| | | | | | | | Tr. | 61,99 | 5,23 | 3,76 |
| 300872 | 3-Cl | (S, S) | + 39,5° | $C_{18}H_{18}ClNO_4$ | 347,789 | 90 | Cal. | 62,16 | 5,22 | 4,03 |
| | | | | | | | Tr. | 61,99 | 5,28 | 3,88 |
| 340176 | 3-Cl | (R, S) | - 16,2° | $C_{18}H_{18}ClNO_4$ | 347,789 | 78 | Cal. | 62,16 | 5,22 | 4,03 |
| | | | | | | | Tr. | 61,85 | 5,41 | 3,78 |
| 340177 | 3-Cl | (S, R) | + 16,6° | $C_{18}H_{18}ClNO_4$ | 347,789 | 80 | Cal. | 62,16 | 5,22 | 4,03 |
| | | | | | | | Tr. | 62,77 | 5,48 | 4,09 |

TABLEAU 2

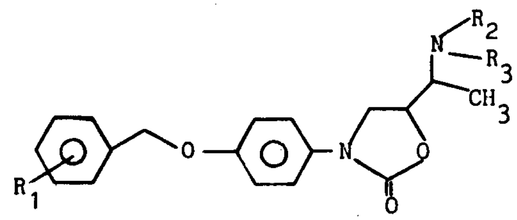

(I)

| Numéro de Code | $R_1$ | $R_2$ | $R_3$ | Configuration | $[\alpha]_D^{20}$ | Formule brute | Poids moléculaire | Point de fusion (°C) | ANALYSE ELEMENTAIRE % | C | H | N |
|---|---|---|---|---|---|---|---|---|---|---|---|---|
| 340271 | 3-Cl | $CH_3$ | H | (R,S) | - 66° (C=1 ; $H_2O$)[a] | $C_{19}H_{21}N_2O_3Cl$ | 360,861[a] | 94[b] | Cal. | 57,44 | 5,58 | 7,05[a] |
| | | | | | | | | 209[a] | Tr. | 56,85 | 5,32 | 6,97 |
| 340179 | " | $CH_3$ | " | (S,R) | +31,1°(C=1 ; $CH_2Cl_2$)[b] +59,8°(C=1 ; $CH_3OH$)[a] | $C_{19}H_{21}ClN_2O_3$ | 360,831[b] | 91[b] | Cal. | 57,44 | 5,58 | 7,05[a] |
| | | | | | | | | 198[a] | Tr. | 57,10 | 5,79 | 7,53 |
| 340276 | " | $CH_3$ | " | (R,R) | -35,4°(C=0,8;$CH_2Cl_2$)[b] | $C_{19}H_{21}N_2O_3Cl$ | 360,861[b] | 220[a] | Cal. | 57,44 | 5,58 | 7,05[a] |
| | | | | | | | | | Tr. | 57,20 | 5,61 | 7,05 |
| 340333 | " | $CH_3$ | " | (S,S) | +44,4°(C=1;$CH_2Cl_2$)[b] | $C_{19}H_{21}ClN_2O_3$ | 360,831[b] | 85[b] | Cal. | 57,44 | 5,58 | 7,05[a] |
| | | | | | | | | 225[a] | Tr. | 57,30 | 5,86 | 7,18 |

TABLEAU 2 (suite)

| Numéro de Code | $R_1$ | $R_2$ | $R_3$ | Configuration | $[\alpha]_D^{20}$ | Formule brute | Poids moléculaire | Point de fusion (°C) | ANALYSE ELEMENTAIRE % | C | H | N |
|---|---|---|---|---|---|---|---|---|---|---|---|---|
| 200562 | 3-Cl | $CH_3$ | $CH_3$ | (R,S) | $-34,7°(C=1;CH_2Cl_2)^b$<br>$-29,7°(C=1;CH_2Cl_2)^a$ | $C_{20}H_{23}ClN_2O_3$<br>$C_{20}H_{24}Cl_2N_2O_3$<br>+2,6% $H_2O$ | 374,857[b]<br>422,302[a] | 124[b]<br>117[a] | Cal. | 56,88 | 6,02 | 6,63[a] |
| | | | | | | | | | Tr. | 56,79 | 5,84 | 6,53 |
| 340321 | " | H | H | (R,R) | $-38,3°(C=1;CH_2Cl_2)^b$ | $C_{18}H_{19}ClN_2O_3$ | 346,805[b] | 99 | Cal. | 55,10 | 5,39 | 7,14[a] |
| | | | | | | | | | Tr. | 55,11 | 5,11 | 7,06 |
| 340366 | " | " | " | (S,S) | $+41°(C=1;CH_2Cl_2)^b$ | $C_{18}H_{19}ClN_2O_3$ | 346,805[b] | 106[b]<br>220[a] | Cal. | 56,40 | 5,26 | 7,31[a] |
| | | | | | | | | | Tr. | 56,13 | 5,19 | 7,12 |
| 340742 | 3-Cl | $CH_3$ | $CH_3$ | (R*,S*)<br>(±)[e] | (±)[e] | $C_{20}H_{23}ClN_2O_3$<br>$C_{20}H_{24}Cl_2N_2O_3$<br>+ 1,5 $H_2O$ | 374,857<br>438,346 | 114[b]<br>86-90[a] | Cal. | - | - | - |
| | | | | | | | | | Tr. | - | - | - |

TABLEAU 2 (suite)

| Numéro de Code | $R_1$ | $R_2$ | $R_3$ | Configuration | $[\alpha]_D^{20}$ | Formule brute | Poids moléculaire | Point de fusion (°C) | ANALYSE ELEMENTAIRE % | C | H | N |
|---|---|---|---|---|---|---|---|---|---|---|---|---|
| 340743 | 3-$OCH_3$ | $CH_3$ | $CH_3$ | (R*,S*) (±)[e] | (±)[e] | $C_{21}H_{26}N_2O_4$<br>$C_{25}H_{30}N_2O_8$ | 370,434<br>486,506 | huile[b]<br>132[c] | Cal. | - | - | - |
| | | | | | | | | | Tr. | - | - | - |
| 340744 | 3-$OCH_3$ | $CH_3$ | $CH_3$ | (R, S) | -47,5° (C=0,08; $CH_3OH$)[b]<br>-40° (c=0,1; EtOH à 95%) | $C_{21}H_{26}N_2O_4$<br>$C_{25}H_{30}N_2O_8$ | 370,434<br>486,506 | 62-64[b]<br>126-130[c] | Cal. | - | - | - |
| | | | | | | | | | Tr. | - | - | - |
| 340745 | 3-Cl | H | $CH_3$ | (R*,S*) (±)[e] | (±)[e] | $C_{19}H_{21}ClN_2O_3$<br>$C_{19}H_{22}Cl_2N_2O_3$ | 360,831<br>397,296 | 102[b]<br>196[a] | Cal. | - | - | - |
| | | | | | | | | | Tr. | - | - | - |
| 340746 | 3-Cl | $CH_3$ | $CH_3$ | (S, R) | +28° (c=2; $CH_3OH$)[b]<br>+47° (c=0,6; $CH_3OH$)[d] | $C_{20}H_{23}ClN_2O_3$<br>$C_{24}H_{27}ClN_2O_7$ | 374,857<br>490,929 | -<br>150[d] | Cal. | - | - | - |
| | | | | | | | | | Tr. | - | - | - |

TABLEAU 2 (suite)

| Numéro de Code | $R_1$ | $R_2$ | $R_3$ | Configuration | $[\alpha]_D^{20}$ | Formule brute | Poids moléculaire | Point de fusion (°C) | ANALYSE ELEMENTAIRE % | C | H | N |
|---|---|---|---|---|---|---|---|---|---|---|---|---|
| 340747 | 3-$OCH_3$ | $CH_3$ | $CH_3$ | (S, R) | +49°(c=0,06;EtOH à 95%)[b]<br>+37°(c=0,1;EtOH à 95%)[c] | $C_{21}H_{26}N_2O_4$<br>$C_{25}H_{30}N_2O_8$ | 370,434<br>486,506 | 63[b]<br>126[c] | Cal. | - | - | - |
| | | | | | | | | | Tr. | - | - | - |
| 340748 | 3-Cl | $CH_3$ | $CH_3$ | ($R^*$,$R^*$) (±)[e] | (±)[e] | $C_{20}H_{23}ClN_2O_3$<br>$C_{20}H_{24}Cl_2N_2O_3$ | 374,857<br>411,324 | 99[b]<br>200[a] | Cal. | - | - | - |
| | | | | | | | | | Tr. | - | - | - |
| 340749 | 3-$OCH_3$ | $CH_3$ | $CH_3$ | ($R^*$,$R^*$) (±)[e] | (±)[e] | $C_{21}H_{26}N_2O_4$<br>$C_{21}H_{27}ClN_2O_4$ | 370,434<br>406,899 | huile[b]<br>220[a] | Cal. | - | - | - |
| | | | | | | | | | Tr. | - | - | - |
| 340750 | 3-Cl | H | H | ($R^*$,$S^*$) (±) | (±)[e] | $C_{18}H_{19}ClN_2O_3$<br>$C_{18}H_{20}Cl_2N_2O_3$ | 346,805<br>383,270 | 91[b]<br>231[a] | Cal. | - | - | - |
| | | | | | | | | | Tr. | - | - | - |

a = chlorhydrate ; b = base ; c = fumarate ; d= maléate ; e = nomenclature des Chemical Abstracts

TABLEAU 3

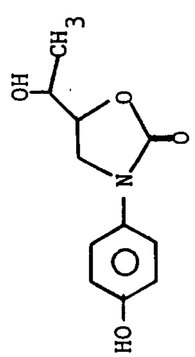

(XI)

| Numéro de Code | Configuration absolue | $[\alpha]_D^{20}$ (C = 1 ; $CH_3OH$) | Formule brute | Poids moléculaire | Point de fusion (°C) | ANALYSE ELEMENTAIRE % | C | H | N |
|---|---|---|---|---|---|---|---|---|---|
| 340 315 | (R, R) | - 73,2° | $C_{11}H_{13}NO_4$ | 223,222 | 149 | Cal. | 59,18 | 5,87 | 6,28 |
| | | | | | | Tr. | 59,42 | 6,11 | 6,13 |
| 200158 | (S, S) | + 71,7° | $C_{11}H_{13}NO_4$ | 223,222 | 154 | Cal. | 59,18 | 5,87 | 6,28 |
| | | | | | | Tr. | 59,42 | 6,11 | 6,13 |
| 200356 | (R, S) | - 28,5° | $C_{11}H_{13}NO_4$ | 223,222 | 163 | Cal. | 59,18 | 5,87 | 6,28 |
| | | | | | | Tr. | 58,88 | 5,73 | 6,12 |
| 200357 | (S, R) | + 29,8° | $C_{11}H_{13}NO_4$ | 223,222 | 163 | Cal. | 59,18 | 5,87 | 6,28 |
| | | | | | | Tr. | 59,14 | 5,85 | 6,09 |

Les composés selon l'invention ont été testés sur l'animal de laboratoire et ont fait preuve d'une activité pharmacologique et notamment une activité inhibitrice de la monoamine oxydase, en particulier de la monoamine oxydase de type B.

Cette activité a été mise en évidence par mise en oeuvre du protocole décrit par DOSTERT P. et coll. dans J. Pharm. Pharmacol., 1983, 35, 161-165 ; ce protocole est basé sur des tests enzymatiques menés

avec ou sans préincubation notamment pendant 60 minutes et il permet de mesurer in vitro l'effet inhibiteur à l'égard de la MAO-A et de la MAO-B du cerveau de rat.

Le tableau 4 ci-après rassemble les concentrations inhibitrices 50 (CI 50) des formes A et B de la MAO, obtenues pour un certain nombre de composés (I) selon l'invention en suivant le protocole ci-dessus appliqué à un homogénat de cerveau total de rat, à 1 g de tissu/16 ml de tampon phosphate de pH 7,4.

TABLEAU 4

| Composé testé No. de code | Configuration | CI 50 (A) en nM | CI 50 (B) en nM | CI 50 (A) / CI 50 (B |
|---|---|---|---|---|
| | | (Préincubation de 60') | | |
| 340271[a] | (R,S) (-) | 7430 | 78 | 95 |
| 340179[a] | (S,R) (+) | 18300 | 1160 | 16 |
| 340276[a] | (R,R) (-) | 148000 | 1350 | 110 |
| 340333[a] | (S,S) (+) | >100000 | 1280 | >78 |
| 200562[a] | (R,S) (-) | >100000 | 53 | >1887 |
| 340321[a] | (R,R) (-) | >100000 | 5340 | >19 |
| 340366[a] | (S,S) (+) | >100000 | 1641 | >61 |
| 340742[a] | (R*,S*) (±) | >1000000 | 160 | >6250 |
| 340743[c] | (R*,S*)(±) | 1000000 | 400 | 2500 |
| 340744[c] | (R,S) (-) | >1000000 | 250 | >4000 |
| 340745[a] | (R*,S*)(±) | 15000 | 50 | 300 |
| 340746[d] | (S,R) (+) | >1000000 | 1600 | >625 |
| 340747[a] | (S,R) (+) | >1000000 | 15000 | >67 |
| 340748[a] | (R*,R*)(±) | 300000 | 20000 | >15 |
| 340749[a] | (R*,R*)(±) | >300000 | 30000 | >10 |
| 340750 | (R*,S*)(±) | 210000 | 380 | 553 |

a = chlorhydrate ; c = fumarate ; d = maléate

Concentrations de substrat : [$^{14}$C] 5-HT = 480 µM [$^{14}$C] PEA = 12 µM pour l'étude de l'inhibition des formes A et B.

Il ressort de ce tableau 4 que les composés (I) selon l'invention présentent une activité inhibitrice de la MAO et en particulier que les composés de configuration érythro (R,S) (-) ou (R*, S*) (±) sont des inhibiteurs puissants et selectifs de la MAO de type B.

Par ailleurs, une étude toxicologique sub-aigüe 15 jours effectuée sur le rat a revélé l'inocuité des composés (I) selon l'invention et de leurs sels d'addition d'acide pharmaceutiquement acceptables.

Il découle de ce qui précede que les composés (I) et leurs sels d'addition d'acide pharmaceutiquement acceptables de l'invention trouvent leur application en therapeutique, notamment en tant que médicaments inhibiteurs de la monoamine oxydase, en particulier de la monoamine oxydase de type B. Ces composés et sels pourront donc être utilisés pour la préparation d'un médicament pour le traitement des dépressions, de la maladie de Parkinson et des déficits neurologiques notamment liés à la sénescence.

L'invention s'étend aux compositions pharmaceutiques renfermant, à titre de principe actif, au moins un composé choisi parmi les composés (I) et leurs sels d'addition d'acide pharmaceutiquement acceptables selon l'invention, en association avec un véhicule pharmaceutiquement acceptable. Ces compositions seront administrées par voie orale sous forme de comprimés, dragées ou gélules par exemple, à des doses de principe actif pouvant aller jusqu'à 50 mg/jour en une ou plusieurs prises, par voie rectale sous forme de suppositoires contenant jusqu'à 300 mg de principe actif (1 à 2 par jour) ou encore sous forme de solutés injectables contenant jusqu'à 300 mg de principe actif (1 à 2 injections par jour).

## Revendications

**Revendications pour les Etats contractants suivants : BE, CH/LI, DE, FR, GB, IT, LU, NL, SE**

**1.** Dérivés 5-aminoéthylés de l'oxazolidinone-2, caractérisés en ce qu'ils sont choisis dans l'ensemble constitué par :

(i) les composés de formule

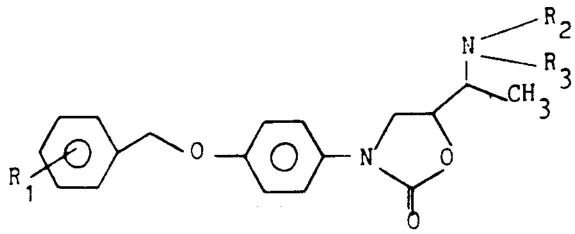

[IA(-)]

dans laquelle $R_1$ représente :

- un atome d'hydrogène,
- un groupe alkoxy comportant de 1 à 4 atomes de carbone,
- un groupe trifluorométhyle, ou
- un ou deux atomes d'halogène ;

et le couple ($R_2$, $3_3$) représent (H, H), (H, alkyle en $C_1$-$C_4$) ou (alkyle en $C_1$-$C_4$, alkyle en $C_1$-$C_4$),ces composés étant de configuration érythro (R, S) (-) ;

(ii) les racemiques de configuration (R*, S*) (±) comprenant chacun un composé [IA(-)] tel que défini en (i) et l'énantiomère de configuration érythro (S, R) (+) [IA(+)] de ce composé ; et

(iii) les sels d'addition d'acide organique ou minéral des composés définies en (i) et des racémiques définis en (ii).

**2.** Dérivés selon la revendication 1 pour lesquels $R_1$ représente un atome de chlore ou un groupe méthoxy, en position méta, et le couple ($R_2$, $R_3$) représente ($CH_3$, $CH_3$).

**3.** Composition pharmaceutique, à activité inhibitrice de la monoamine oxydase, caractérisée en ce qu elle comprend un dérivé selon l'une des revendications 1 et 2, en association avec un véhicule pharmaceutique acceptable.

**4.** Procédé de préparation des dérivés de formule [IA(-)] et des dérivés de formule [IA(+)] selon la revendication 1, ainsi que leurs sels d'addition d'acide, caractérisé en ce qu'il consiste :

- à condenser un composé de formule :

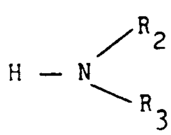

(X)

où le couple ($R_2$, $R_3$) a la même signification que dans la formule [IA(-)] définie à la revendication 1, respectivement avec les stéréoisomères mésylates thréo (-) [IXB(-)] et thréo (+) [IXB(+)] correspondant à la formule :

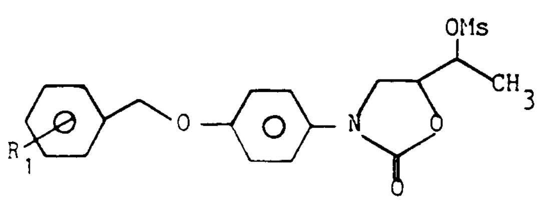

(IX)

où Ms représente le groupe mésyle et $R_1$ a la même signification que dans la revendication 1, puis éventuellement :

- à former les sels d'addition d'acide des composés ainsi obtenus.

**5.** Procédé selon la revendication 4, caractérisé en ce que la réaction entre les composés X et IX est effectuée dans un solvant hydroxylé.

**6.** Procédé selon la revendication 4 ou 5, caractérisé en ce que les stéréoisomères mésylates de formule (IX) sont obtenus par action d'un halogénure de mésyle respectivement sur les stéréoisomères thréo (+) [VIB(+)] et thréo (-) (VIB(-)] correspondant à la formule :

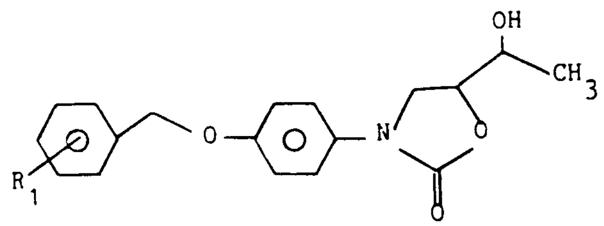

(VI)

où $R_1$ a la même signification que dans la revendication 1.

**7.** Procédé selon la revendication 6, caractérisé en ce que la réaction est effectuée en présence d'une base organique.

**8.** Stéréoisomères mésylates thréo (+) [IXB(+)] et thréo (-) (IXB(-)] correspondant à la formule :

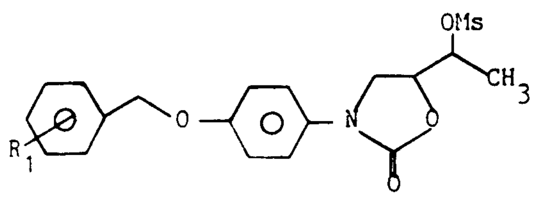

(IX)

où Ms représente le groupe mésyle et $R_1$ a la même signification que dans la revendication 1.

**Revendications pour les Etats contractants suivants : ES, GR**

**1.** Procédé de préparation des composés érythro (S, R) (+) [IA(+)] et érythro (R, S) (-) [IA(-)], correspondant à la formule :

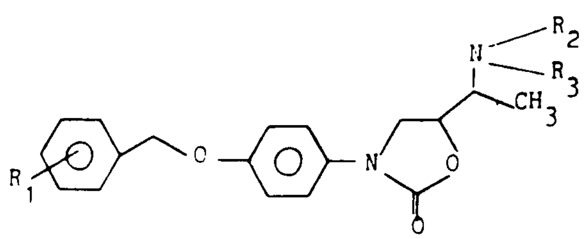

(I)

dans laquelle $R_1$ représente :

- un atome d'hydrogène,
- un groupe alkoxy comportant de 1 a 4 atomes de carbone,
- un groupe trifluorométhyle, ou
- un ou deux atomes d'halogène :

et le couple ($R_2$, $R_3$) représente (alkyle en $C_1$-$C_4$, alkyle en $C_1$-$C_4$), (H, H) ou (H, alkyle en $C_1$-$C_4$), ainsi

que de leurs sels d'addition d'acide,
caractérisé en ce qu'il consiste :

- à condenser un composé de formule :

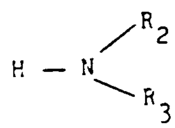

(X)

où le couple ($R_2$, $R_3$) a la même signification que ci-dessus, respectivement avec les stéréoisomères mésylates thréo (+) [IXB(+)] et thréo (-) [IXB(-)] correspondant à la formule : la formule :

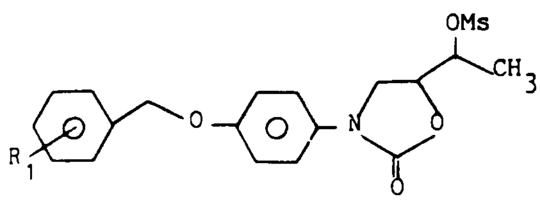

(IX)

où Ms représente le groupe mésyle et $R_1$ a la même signification que ci-dessus, puis éventuellement

- à former les sels d'addition d'acide des composés ainsi obtenus.

2. Procédé selon la revendication 1, caractérisé en ce que la réaction entre les composés (X) et (IX) est effectuée dans un solvant hydroxylé.

3. Procédé selon la revendication 1 ou 2, caractérisé en ce que les stéréoisomères mésylates de formule (IX) sont obtenus par action d'un halogénure de mésyle respectivement sur les stéréoisomères thréo (+) [VIB(+)] et thréo (-) [VIB(-)] correspondant à la formule :

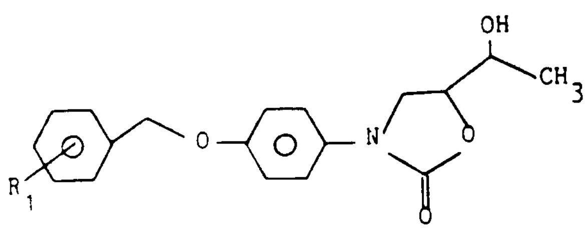

(VI)

où $R_1$, a la même signification que dans la formule (I) définie à la revendication 1.

4. Procédé selon la revendication 3, caractérisé en ce que la réaction est effectuée en présence d'une base organique.

5. Procédé de préparation d'une composition pharmaceutique ayant une activité inhibitrice de la monoamine oxydase, caractérisé en ce qu'il consiste à mélanger un véhicule pharmaceutiquement acceptable avec le composé érythro (R, S) (-) [IA(-)] ou le mélange racémique des composés [IA(-)] et [IA(+)], ces composés correspondant à la formule (I) définie à la revendication 1.

**Claims**

**Claims for the following Contracting States : BE, CH/LI, DE, FR, GB, IT, LU, NL, SE**

1. 5-aminoethyl oxazolidin-2-one derivatives, characterized in that they are chosen among:
(i) the compounds of formula:

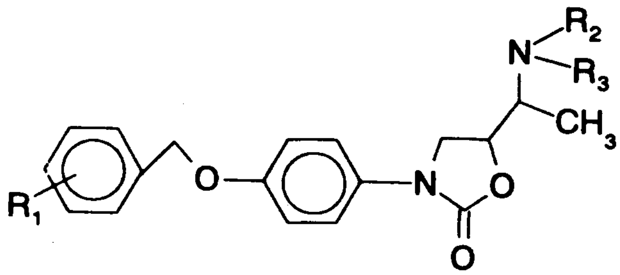

[IA(-)]

wherein $R_1$ represents:
- a hydrogen atom,
- a $C_1$-$C_4$ alkoxy group,
- a trifluoromethyl group, or
- one or two halogen atoms;

and the pair ($R_2$, $R_3$) is (H, H), (H, $C_1$-$C_4$ alkyl) or ($C_1$-$C_4$ alkyl, C,-$C_4$ alkyl), these compounds being of erythro (R, S) (-) configuration;

(ii) the racemates of ($R^*$, $S^*$) (±) configuration each comprising a compound [IA(-)] as defined under (i) and the enantiomer [IA(+)] of erythro (S, R) (+) configuration of this compound; and

(iii) the mineral or organic acid addition salts of the compounds defined under (i) and of the racemates defined under (ii).

**2.** Derivatives according to claim 1, for which $R_1$ is a chlorine atom or methoxy group, in meta position, and the couple ($R_2$, $R_3$) is ($CH_3$, $CH_3$).

**3.** A pharmaceutical composition, acting as an inhibitor of monoamine oxidase, characterized in that it comprises a derivative according to any of claims 1 and 2, in association with a pharmaceutically acceptable carrier.

**4.** A process for preparing the derivatives of formula [IA(-)] and the derivatives of formula [IA(+)] according to claim 1, as well as their acid addition salts, characterized in that it consists:
- in condensing a compound of formula:

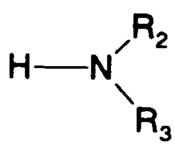

(X)

where the pair ($R_2$, $R_3$) has the same meaning as in formula [IA(-)] defined in claim 1, respectively with the threo (-) mesylate stereoisomers [IXB(-)] and the threo (+) mesylate stereoisomers [IXB(+)] corresponding to the formula:

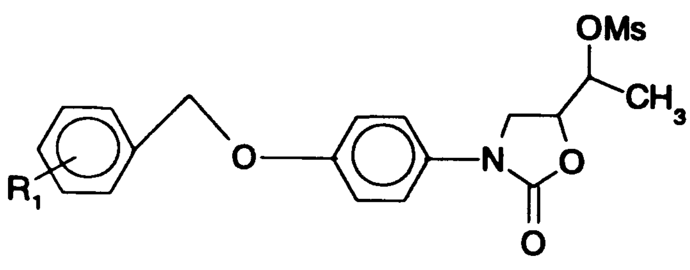

(IX)

where Ms represents mesyl and $R_1$ has the same meaning as in claim 1, then optionally:

- in forming the acid addition salts of the compounds thus obtained.

**5.** A process according to claim 4, characterized in that the reaction between the compounds (X) and (IX) is effected in a hydroxylated solvent.

**6.** A process according to claim 4 or 5, characterized in that mesylate stereoisomers of formula (IX) are obtained by the action of a mesyl halide respectively on the threo (+) stereoisomers [VIB(+)] and the threo (-) stereoisomers [VIB(-)] corresponding to the formula:

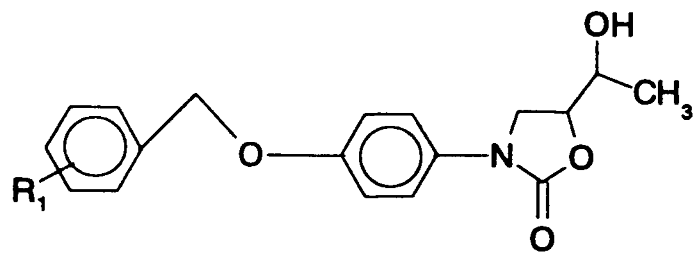

(VI)

where $R_1$ has the same meaning as in claim 1.

**7.** A process according to claim 6, characterized in that the reaction is carried out in the presence of an organic base.

**8.** Threo (+) mesylate stereoisomers [IXB(+)] and threo (-) mesylate stereoisomers [IXB(-)] corresponding to the formula:

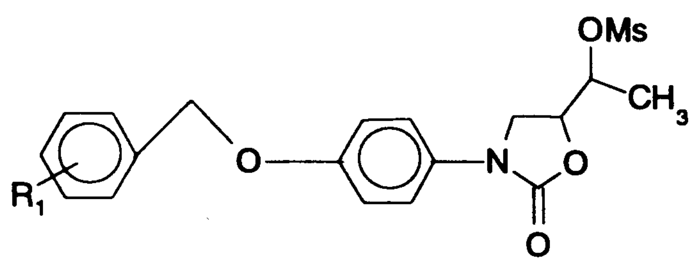

(IX)

where Ms represents mesyl and $R_1$ has the same meaning as in claim 1.

**Claims for the following Contracting States : ES, GR**

**1.** Process for preparing the erythro (S, R) (+) compounds [IA(+)] and the erythro (R, S) (-) compounds [IA(-)] corresponding to formula:

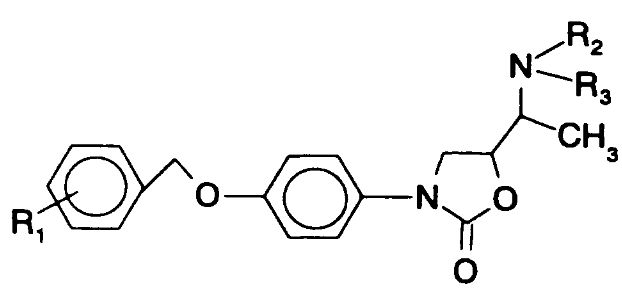

(I)

wherein $R_1$ represents:
- a hydrogen atom,
- a $C_1$-$C_4$ alkoxy group,

- a trifluoromethyl group, or
- one or two halogen atoms;

and the pair ($R_2$, $R_3$) is (H, H), (H, $C_1$-$C_4$ alkyl) or ($C_1$-$C_4$ alkyl, $C_1$-$C_4$ alkyl), as well as the acid addition salts thereof, characterized in that it consists:

- in condensing a compound of formula:

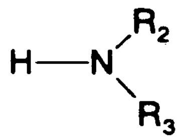

(X)

where the pair ($R_2$, $R_3$) has the same meaning as above, respectively with the threo (+) mesylate stereoisomers [IXB(+)] and the threo (-) mesylate stereoisomers [IXB(-)] corresponding to the formula:

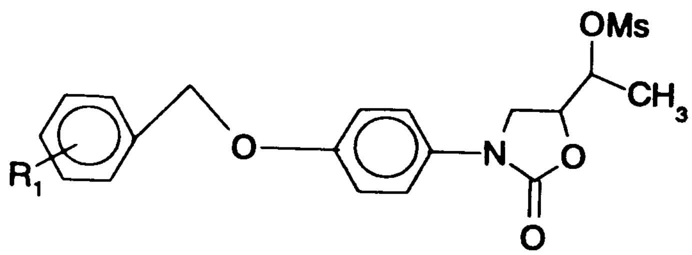

(IX)

where Ms represents mesyl and $R_1$ has the same meaning as above, then optionally:

- in forming the acid addition salts of the compounds thus obtained.

**2.** A process according to claim 1, characterized in that the reaction between the compounds (X) and (IX) is effected in a hydroxylated solvent.

**3.** A process according to claim 1 or 2, characterized in that mesylate stereoisomers of formula (IX) are obtained by the action of a mesyl halide respectively on the threo (+) stereoisomers [VIB(+)] and the threo (-) stereoisomers [VIB(-)] corresponding to the formula:

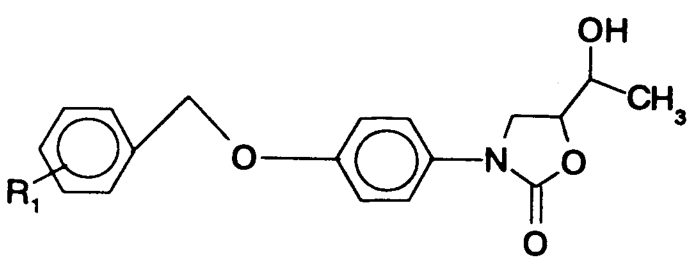

(VI)

where $R_1$ has the same meaning as in formula (I) defined in claim 1.

**4.** A process according to claim 3, characterized in that the reaction is carried out in the presence of an organic base.

**5.** A process for preparing a pharmaceutical composition acting as an inhibitor of monoamine oxidase, characterized in that it consists in mixing a pharmaceutically acceptable carrier with the erythro (R, S) (-) compound [IA(-)] or the racemic mixture of compounds [IA(-)] and [IA(+)], said compounds corresponding to formula (I) defined in claim 1.

**Patentansprüche**
**Patentansprüche für folgende Vertragsstaaten : BE, CH/LI, DE, FR, GB, IT, LU, NL, SE**

1. Oxazolidin-2-on-5-aminoethyl-Derivate, dadurch gekennzeichnet, daß sie aus der aus

(i) den Verbindungen der Formel

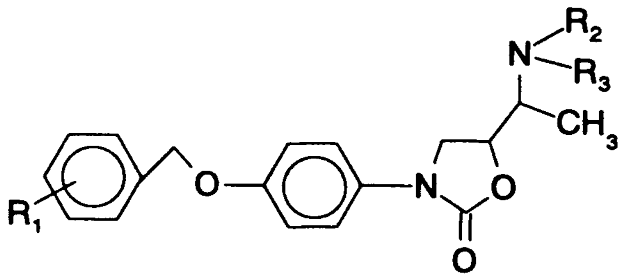

[IA(-)]

worin $R_1$

- ein Wasserstoff-Atom,
- eine 1 bis 4 Kohlenstoff-Atome enthaltende Alkoxy-Gruppe,
- eine Trifluormethyl-Gruppe oder
- ein oder zwei Halogenatome bedeutet;

und die Paarung ($R_2$, $R_3$) (H, H), (H, $C_1$-$C_4$ Alkyl) oder ($C_1$-$C_4$ Alkyl, $C_1$-$C_4$ Alkyl) bedeutet, wobei diese Verbindungen die erythro-(R,S)(-)-Konfiguration haben;

(ii) den Racematen mit der (R*,R*)(±)-Konfiguration, welches jedes eine Verbindung [IA(-)] wie in (i) definiert und das Enantiomer dieser Verbindung mit der erythro(S,R)(+)-Konfiguration (IA(+)] enthalten, und

(iii) den Additionssalzen der in (i) definierten Verbindungen und der in (ii) definierten Racemate mit organischen oder Mineralsäuren bestehenden Gruppe ausgewählt sind.

2. Derivate gemäß Anspruch 1, in welchen $R_1$ ein Chlor-Atom oder eine Methoxy-Gruppe in meta-Stellung bedeutet und die Paarung ($R_2$, $R_3$) ($CH_3$, $CH_3$) bedeutet.

3. Pharmazeutische Zusammensetzung mit monoaminoxydasehemmender Wirksamkeit, dadurch gekennzeichnet, daß sie ein Derivat gemäß einem der Ansprüche 1 und 2 in Verbindung mit einem pharmazeutisch annehmbaren Träger enthält.

4. Verfahren zur Herstellung von Derivaten der Formel [IA(-)] und von Derivaten der Formel [IA(+)] gemäß Anspruch 1 als auch ihrer Säureadditionssalze, dadurch gekennzeichnet, daß es aus

- dem Kondensieren einer Verbindung der Formel

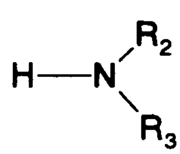

(X)

worin die Paarung ($R_2$, $R_3$) dieselbe Bedeutung wie in der in Anspruch 1 definierten Formel [IA(-)] hat, mit den der Formel

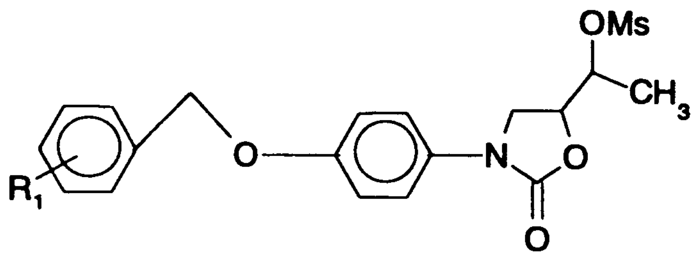

(IX)

entsprechenden stereoisomeren Mesylaten threo-(-)-[IXB(-)] beziehungsweise threo-(+)-[IXB(+)], worin Ms die Mesyl-Gruppe bedeutet und $R_1$ dieselbe Bedeutung wie in Anspruch 1 hat, und darauf gegebenenfalls

- dem Bilden der Säureadditionssalze der auf diese Weise erhaltenen Verbindungen besteht.

5. Verfahren gemäß Anspruch 4, dadurch gekennzeichnet, daß die Reaktion zwischen den Verbindungen X und IX in einem hydroxylhaltigen Lösungsmittel durchgeführt wird.

6. Verfahren gemäß Anspruch 4 oder 5, dadurch gekennzeichnet, daß die stereoisomeren Mesylate der Formel (IX) durch Einwirkung eines Mesylhalogenids auf die der Formel

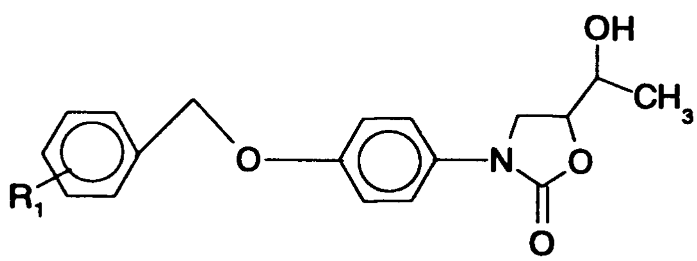

(VI)

entsprechenden Stereoisomeren threo-(+)-[VIE(+)] beziehungsweise threo-(-)-[VIB(-)], worin $R_1$ dieselbe Bedeutung wie in Anspruch 1 hat, erhalten werden.

7. Verfahren gemäß Anspruch 6, dadurch gekennzeichnet, daß die Reaktion in Gegenwart einer organischen Base durchgeführt wird.

8. Der Formel

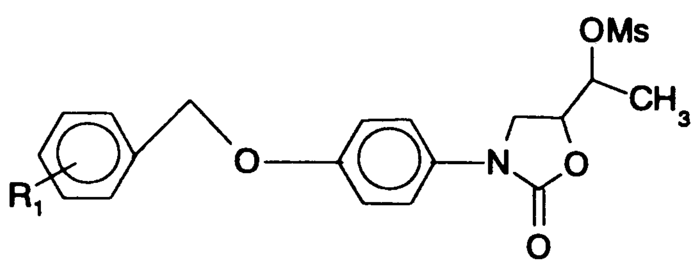

(IX)

entsprechende stereoisomere Mesylate threo-(+)-[IXB(+)] und threo-(-)-[IXB(-)], worin Ms die Mesyl-Gruppe bedeutet und $R_1$ dieselbe Bedeutung wie in Anspruch 1 hat.

**Patentansprüche für folgende Vertragsstaaten : ES, GR**

1. Verfahren zur Herstellung der der Formel

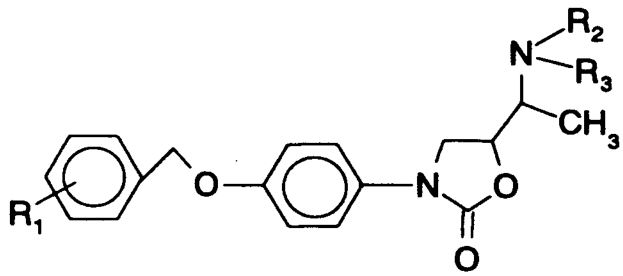

(I)

entsprechenden Verbindungen erythro-(S,R)(+)-[IA(+)] und erythro-(R,S)(-)-[IA(-)], worin $R_1$

- ein Wasserstoff-Atom,
- eine 1 bis 4 Kohlenstoff-Atome enthaltende Alkoxy-Gruppe,
- eine Trifluormethyl-Gruppe oder
- ein oder zwei Halogenatome bedeutet;

und die Paarung ($R_2$, $R_3$) ($C_1$-$C_4$ Alkyl, $C_1$-$C_4$ Alkyl), (H, H>, oder (H, $C_1$-$C_4$ Alkyl) bedeutet, als auch ihrer Säureadditionssalze, dadurch gekennzeichnet, daß es aus

- dem Kondensieren einer Verbindung der Formel

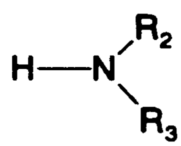

(X)

worin die Paarung ($R_2$, $R_3$) dieselbe Bedeutung wie voranstehend hat, mit den der Formel

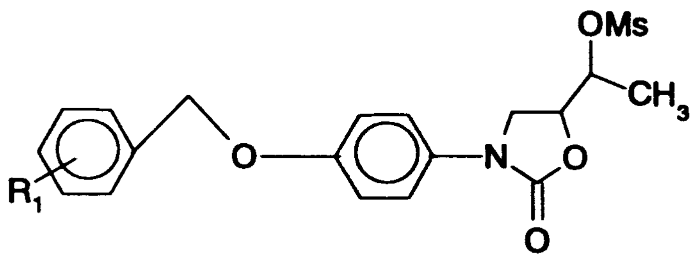

(IX)

entsprechenden stereoisomeren Mesylaten threo-(+)-[IXB(+>)] beziehungsweise threo-(-)-[IXB(-)], worin Ms die Mesyl-Gruppe bedeutet und $R_1$ dieselbe Bedeutung wie voranstehend hat, und darauf gegebenenfalls

- dem Bilden der Säureadditionssalze der auf diese Weise erhaltenen Verbindungen besteht.

**2.** Verfahren gemäß Anspruch 1 dadurch gekennzeichnet, daß die Reaktion zwischen den Verbindungen (X) und (IX) in einem hydroxylhaltigen Lösungsmittel durchgeführt wird.

**3.** Verfahren gemäß Anspruch 1 oder 2, dadurch gekennzeichnet, daß die stereoisomeren Mesylate der Formel (IX) durch Einwirkung eines Mesylhalogenids auf die der Formel

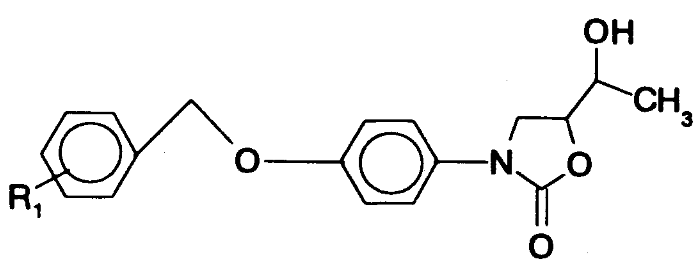

(VI)

entsprechenden Stereoisomeren threo-(+)-[VIB(+)] beziehungsweise threo-(-)-[VIB(-)], worin $R_1$ dieselbe Bedeutung wie in der in Anspruch 1 definierten Formel (I) hat, erhalten werden.

**4.** Verfahren gemäß Anspruch 3, dadurch gekennzeichnet, daß die Reaktion in Gegenwart einer organischen Base durchgeführt wird.

**5.** Verfahren zur Herstellung einer pharmazeutischen Zusammensetzung mit monoaminoxydasehemmender Wirksamkeit, dadurch gekennzeichnet, daß es aus dem Mischen eines pharmazeutisch annehmbaren Trägers mit der erythro-(R,S)-(-)-Verbindung [IA(-)] oder dem racemischen Gemisch der Verbindungen [IA(-)] und [IA(+)] besteht, wobei diese Verbindungen der in Anspruch 1 definierten Formel (I) entsprechen.